Europäisches Patentamt

European Patent Office

Office européen des brevets

Veröffentlichungsnummer: **0 295 358**

**A1**

# EUROPÄISCHE PATENTANMELDUNG

Anmeldenummer: 87890137.0

Int. Cl.4 **C08B 30/04**

Anmeldetag: **16.06.87**

Veröffentlichungstag der Anmeldung:
**21.12.88 Patentblatt 88/51**

Benannte Vertragsstaaten:
**AT BE DE ES FR GB IT NL SE**

Anmelder: **Österreichische Agrar-Industrie Gesellschaft m.b.H.**
**Hollandstrasse 2**
**A-1020 Wien(AT)**

Anmelder: **Westfalia Separator AG**
**Werner-Habig-Strasse 1 Postfach 3720**
**D-4740 Oelde 1(DE)**

Erfinder: **Marihart, Johann, Dipl.-Ing.**
**Conrathstrasse 5**
**A-3952 Gmünd(AT)**
Erfinder: **Huster, Heinrich, Obering**
**Friedrich-Wilhelm-Weber-Strasse 6**
**D-4740 Oelde 1(DE)**

Vertreter: **Atzwanger, Richard, Dipl.-Ing.**
**Mariahilfer Strasse 1c**
**A-1060 Wien(AT)**

Verfahren zum Aufschliessen stärkehaltiger Rohstoffe.

Bei einem Verfahren zum Aufschließen von stärkehaltigen Rohstoffen, wie Körnerfrüchten, um Fermentationssubstrate zu gewinnen, wird der Rohstoff unter einem Druck von 5 bar bis 10 bar in einem Wasser und bzw. oder rückgeführte Schlempe enthaltenden Medium bei einer Temperatur unterhalb des Stärkeverkleisterungspunktes, insbesondere bei einer Temperatur zwischen 10°C und 60°C, quellen gelassen, bis der Wassergehalt des gequollenen Rohstoffes 45% bis 50% beträgt. Der Quellvorgang kann dabei in Gegenwart von Enzymen, wie α-Amylase oder von uns Pilzen oder Bakterien gewonnener Amyloclucosidase und bzw. oder von cellulolytischen, pektolytischen oder proteolytischen Enzymen ausgeführt werden.

## Verfahren zum Aufschließen stärkehaltiger Rohstoffe

Die Erfindung betrifft ein Verfahren zum Aufschließen von stärkehaltigen Rohstoffen zur Gewinnung von Fermentationssubstraten.

Bekannte Aufschlußverfahren für stärkehaltige Rohstoffe zur Verflüssigung und Verzuckerung des Kohlehydratanteiles als Ausgangssubstrat für mikrobiologische Prozesse, z.B. der Vergärung zu Alkohol, zu Milschsäure, zu Zitronensäure usw. und zur Verhefung, sind das HenzeDämpfverfahren und der Auschluß mittels Jet-Kocher oder mittels Stiftmühlen. Dabei werden am Ende des Aufschlußverfahrens oder nach diesem Enzyme beigesetzt.

Diese bekannten Schlemperecyclingverfahren bedingen jedoch deshalb Viskositätsprobleme, da Hemicellulose-und Pektinanreicherungen und eine nicht ausreichende Durchquellung in den äußeren Randschichten des Stärkerohstoffes zur Ausbildung einer Sperrschicht aus verkleisteter Stärke führen. Wenn hingegen während des Aufschließens die Temperatur erhöht wird, um eine zu hohe Viskosität zu vermeiden, dann tritt die Stärkeverkleisterung schon vor der Aufschlußmaschine ein und ist eine Durchweichung mit rückgeführter Schlempe überhaupt nicht mehr möglich. Bei den bekannten Maischverfahren führt dies zu einem Mehraufwand an Aufschlußenergie und zu einem Mehraufwand an Enzymen.

Eine besondere Vorbehandlung, wie eine intensive Durchquellung der Rohstoffe unter derjenigen Temperatur, bei welcher eine Stärkeverkleisterung eintritt, ist deshalb nicht üblich, da für eine intensive Durchquellung der gebräuchlichen Rohstoffe mindestens zwölf Stunden Quellzeit benötigt werden. Neben hohen Investitionskosten für eine Quellanlage ist außerdem die unerwünschte Anwendung von Hilfsstoffen zu Koservierungszwecken, z.B. Schwefeldioxid, erforderlich.

In der EP-A-60 380 ist im Zusammenhang mit einem Verfahren zur Gewinnung von Stärke im Naßverfahren eine Verkürzung der Quellzeit von Getreide durch Anwendung von erhöhtem hydraulischen Druck vorgeschlagen worden. Bei diesem bekannten Verfahren wird bei der Kurzzeitquellung unter Druck auf eine gezielte chemische, biochemische oder mikrobielle Einflußnahme auf die Rohstoffe verzichtet, da die für die Stärkegewinnung maßgebliche Behandlung der Getreidekornstruktur auf mechanischem Wege in einer besonderen Hochdruckapparatur erfolgt. Bei diesem bekannten Verfahren ist die Quellung unter Druck im Hinblick auf die für die weitere Behandlung des Getreides notwendige Wasseraufnahme von Bedeutung. Über die Anwendung und Möglichkeiten der Quellung von Getreide unter Druck in einem Aufschlußverfahren für stärkehaltige Rohstoffe zur Verwendung als Fermentationssubstrat ergeben sich jedoch aus dieser Literaturstelle keine Anhaltspunkte.

Der Erfindung liegt demgegenüber die Aufgabe zugrunde, ein Aufschlußverfahren für stärkehaltige Rohstoffe durch Einsparungen an mechanischer Aufschlußenergie und Hilfsstoffen unter Steigerung der Substratausbeute einfacher und wirtschaftlicher zu gestalten. Dies wird erfindungsgemäß dadurch erzielt, daß der stärkehaltige Rohstoff unter Druck bei Temperaturen unterhalb dem Stärkeverkleisterungspunkt in Wasser und bzw. oder rückgeführtem Fermentationsrückstand aufgequollen wird. Vorzugsweise erfolgt der Quellvorgang in Gegenwart von Enzymen. So kann der stärkehaltige Rohstoff unter einem Druck von 5 bar bis 10 bar in Wasser bzw. rückgeführtem Fermentationsrückstand (Schlempe) gequollen werden. Bei diesem Verfahren dringt das Quellmedium durch den erhöhten Druck über Hohlräume und Risse in den körnigen Rohstoff ein und kann es somit diesen rasch und vollständig penetrieren. Die notwendige Quellzeit wird durch die mögliche Diffusionsgeschwindigkeit des Quell mediums bestimmt und hängt in erster Linie vom aufzuschließenden Rohstoff und zudem vom gewünschten Ausmaß der enzymatischen Reaktionen in den Getreidekörnern ab.

Vorzugsweise beträgt die Quelltemperatur 60° C bis 70° C. Bei den meisten Getreidearten wird bei Anwendung des erfindungsgemäßen Verfahrens bei 2 bis 5 Stunden Verweilzeit ein Feuchtgehalt von 45% bis 50% erreicht.

Bei der Verarbeitung eines erfindungsgemäß gequollenen Rohstoffes ist der nachfolgende mechanisch-thermisch-enzymatische Aufschluß z.B. mittels einer aus der US-PS 3 995 838 bekannten Maschine bzw. mit dem in der DE-PS 1 922 932 beschriebenen Verfahren unter wesentlich geringerem Energieaufwand möglich. Weiters kann der erfindungsgemäß aufgeschlossene Rohstoff im Kaltmaischverfahren mit zufriedenstellenden Verzuckerungs-und Endvergärungsgraden unter Verwendung von Pilzamylasen anstelle von temperaturstabilen Bakterienamylasen ausgeführt werden. Die erfindungsgemäße Vorquellung stärkehaltiger Rohstoffe erbringt somit den Vorteil, daß eine Stärkeverflüssigung bei einer Temperatur von 70° C, statt wie bisher bei 95° C bis 100° C erzielt werden kann.

Dieser vorteilhafte Effekt wird durch die Möglichkeit verstärkt, das schon bei der Druckquellung stärkehaltiger Rohstoffe in rückgeführter Schlempe amylolytische, pektolytische, cellulolytische oder proteolytische Enzyme eingesetzt werden. Dadurch können diese Enzyme unter optimalen Temperatur- und pH-Bedinugungen wirken, was in den folgenden Stufen der Vergärung oder Verhefung kaum mehr möglich ist.

Weiters können bei der erfindungsgemäßen Druckquellung, die z.B. vier Stunden lang dauert, wirkungsoptimale Temperatur- und pH-Bedingungen eingestellt werden pektin- bzw. cellulosebedingte Vikositäten abgebaut werden und kann das Korngefüge des stärkehaltigen Rohstoffes durch eindringende Enzyme z.B. solcher mit mittellamellenlösenden Eigenschaften, gelockert werden, wodurch die Einbettungsmatrix der Stärkekörner aufgelockert wird. Zudem kann auch eine optimale Wirkung von Enzymen mit niedrigeren Temperaturoptima erzielt werden, ohne daß sie durch bereits gebildete Glukose oder Maltose - wie dies bei Zusatz erst bei der Verzuckerungsrate der Fall ist - eine Produkthemmung erfahren. Soferne diese Enzyme erst später zugefügt werden, sind die notwendigen pH- und Temperaturbedingungen nicht mehr einstellbar, da dann auf die Hefevitalität und die Nachverzuckerung Rücksicht genommen werden muß.

Das erfindungsgemäße Verfahren ist somit insoferne vorteilhaft, als bei den angewendeten Quellbedingungen Enzyme, z.B.α-Amylase und Glucoamylase, bereits beim Aufschlußverfahren zugegeben werden können, die den körnigen Rohstoff vo₁ ɔenetrieren und dank idealer Verteilung auch bei sparsamerer Dosierung optimal wirken. Zudem eignet sich das erfindungsgemäße Verfahren deshalb besonders für Mehrrohrstoffanlagen, da es eine Reihe von Eingriffsmöglichkeiten durch die Variation von Quellzeit, Druck, Temperatur, Enzymzusatz usw. bietet, wobei die Vorteile des niedrigen Energieverbrauches erhalten bleiben. Da die Quellung unter hydraulischem Druck bei niedrigen Temperaturen erfolgt, ist sie energetisch praktisch ohne negativen Einfluß auf das Verfahren. Vielmehr werden außer den qualitativen Vorteilen auch energetische Vorteile durch den erleichterten thermisch mechanischen und enzymatischen Aufschluß bewirkt. Bei der Verwendung von Mais als Rohstoff ergibt sich durch das erfindungsgemäße Verfahren zudem die Möglichkeit, die wertvollen fett haltigen Maiskeime mit geringem technischen Aufwand separat zu gewinnen.

Nachstehend ist ein nicht beschränkendes Ausführungsbeispiel betreffend den Aufschluß von Roggen zur Verspritung oder Verhefung angeführt:

Roggen wird im Verhältnis 1 : 1 mit Destillationsrückstand der Äthanoldestillation oder Fermentationsrückstand aus der Hefegewinnung nach pH-Einstellung unter Zugabe von Hemicellulose und Pilz-α-Amylase bei 50°C bis 60°C unter einem hydraulischen Druck von 6 bar eingequollen und wird mit einer Verweilzeit von ca. vier Stunden unter diesen Bedingungen gehalten. Beim Austrag aus der Quellapparatur wird der Roggen, der die Flüssigphase praktisch absorbiert hat, einem Aufschluß, vorzugsweise mit Stiftmühlen nach dem Kaltmaischverfahren, zugeführt.

An diesem Beispiel sind die Vorteile des erfindungsgemäßen Verfahrens klar erkannbar. Roggen bedingt durch seinen hohen Hemicelluloseanteil normalerweise große Aufschlußprobleme mit hohen Maischeviskositäten, wodurch deshalb geringe Endvergärungsgrade verursacht werden, da die Stärke infolge der hohen Viskosität schlecht aufgeschlossen wird. Das erfindungsgemäße Verfahren ermöglicht nicht nur, den Rohstoff völlig durchzuquellen, sondern gestattet auch die Zugabe von hemicellulolytischen Enzymen zur Beherrschung der Viskosität bzw. um Viskositätsprobleme durch Gewährleistung der notwendigen Intensität der Einwirkung von α-Amylase und Amyloglucosidase (AMG) erst gar nicht auftreten zu lassen.

## Ansprüche

1. Verfahren zum Aufschließen von stärkehaltigen Rohstoffen zur Gewinnung von Fermentationssubstraten, dadurch gekennzeichnet, daß der stärkehaltige Rohstoff unter Druck bei Temperaturen unterhalb des Stärkeverkleisterungspunktes in Wasser und bzw. oder in rückgeführtem Fermentationsrückstand aufgequollen wird.

2. Verfahren nach Anspruch 1, dadurch gekennzeichnet, daß der Quellvorgang in Gegenwart von Enzymen erfolgt.

3. Verfahren nach einem der Ansprüche 1 und 2, dadurch gekennzeichnet, daß der Quellvorgang bei einem Druck zwischen 2 bar und 50 bar, vorzugsweise zwischen 5 bar und 10 bar, erfolgt.

4. Verfahren nach einem der Ansprüche 1 bis 3, dadurch gekennzeichnet, daß der stärkehaltige Rohstoff 1 bis 10, vorzugsweise 2 bis 5 Stunden, lang gequollen wird.

5. Verfahren nach einem der Ansprüche 1 bis 4, dadurch gekennzeichnet, daß der Quellvorgang bei einer Temperatur zwischen 0°C und 100°C, vorzugsweise zwischen 10°C und 60°C, erfolgt.

6. Verfahren nach einem der Ansprüche 1 bis 5, dadurch gekennzeichnet, daß als stärkehaltigen Rohstoff Körnerfrüchte, wie Roggen, Weisen, Gerste und bzw. oder Mais, eingesetzt werden.

7. Verfahren nach einem der Ansprüche 1 und 6, dadurch gekennzeichnet, daß der Quellvorgang in einem Medium, das 0% bis 100% rückgeführte Schlempe enthält, ausgeführt wird.

8. Verfahren nach einem der Ansprüche 1 und 7. dadurch gekennzeichnet, daß der stärkehaltige Rohstoff bis zu einem Wassergehalt des gequollenen Rohstoffes von 20% bis 60%, vorzugsweise von 45% bis 50%, gequollen wird.

9. Verfahren nach einem der Ansprüche 1 und 8. dadurch gekennzeichnet, daß der Quellvorgang in Gegenwart von $\alpha$-Amylase und von aus Pilzen oder Bakterien gewonnener Amyloclucosidase und bzw. oder von cellulolytischen, pektolytischen oder proteolytischen Enzymen erfolgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 060 380 (WESTFALIA SEPARATA) <br> * Ansprüche * <br> --- | 1-9 | C 08 B 30/04 |
| Y | DE-A-3 211 756 (GG. WESTPHAL) <br> * Ansprüche * <br> --- | 1-9 | |
| A | EP-A-0 204 087 (DR. A.D. HAMMEL) <br> * Ansprüche * <br> --- | | |
| A | CHEMIKER-ZTG, Band 76, Nr. 3, 1952, Seiten 47-50; K.R. DIETRICH: "Kontinuierliche Verzuckerung und Aufschliessung stärkehaltiger Rohstoffe" <br> ----- | | |

| | | | RECHERCHIERTE SACHGEBIETE (Int. Cl.4) |
|---|---|---|---|
| | | | C 08 B |

Der vorliegende Recherchenbericht wurde für alle Patentansprüche erstellt

| Recherchenort | Abschlußdatum der Recherche | Prüfer |
|---|---|---|
| DEN HAAG | 02-03-1988 | LENSEN H.W.M. |